# EUROPEAN PATENT APPLICATION

(11) **EP 4 674 276 A1**
(43) Date of publication of application: **07.01.2026**
(21) Application number: 24185791.1
(22) Date of filing: 01.07.2024
(51) Int. Cl.: A23F 5/48, C11B 1/10, C12P 7/64

(54) **EXTRACTION OF COFFEE OIL/BUTTER FROM FERMENTED SPENT COFFEE GROUNDS USING GREEN SOLVENTS**

(71) Applicant: Helmholtz-Zentrum hereon GmbH, 21502 Geesthacht (DE)
(72) Inventor: Tarazona Lizcano, Natalia Andrea, 10965 Berlin (DE); Machatschek, Rainhard Gabriel, 10965 Berlin (DE)
(74) Representative: Eisenführ Speiser

(57) **Abstract**

The present invention relates to a method for extracting coffee oil from spent coffee grounds, to compositions comprising coffee oil, obtained by or obtainable by such a method, and to cosmetic products comprising such a composition.

## Description

The present invention relates to a method for extracting coffee oil from spent coffee grounds, to compositions comprising coffee oil, obtained by or obtainable by such a method, and to cosmetic products comprising such a composition.

Coffee has become one of the most popular beverages in the world. In the coffee year 2021/2022 (12 months from 1 October to 30 September), approximately 175.6 millions of 60 kg bags of coffee were consumed, with numbers still increasing per year.

With such consumption rates, the residues arising from the coffee consumption cause an increasing impact on the environment. For example, spent coffee grounds, which are obtained after the preparation process of coffee (i.e. the beverage), are typically considered biowaste and contribute to environmental pollution. However, spent coffee grounds still contain residual coffee oil, fats, and other valuable compounds that could be extracted and utilized.

Since a large amount of spent coffee grounds is constantly produced, the extraction of compounds or components from these spent coffee grounds gains increasing attention.

A very efficient extraction method for vegetable oils, including coffee oil from spent coffee grounds, is hexane extraction, which has also been applied to extract coffee oil from spent coffee grounds. The yields of hexane extraction typically range from about 10% to 20% of coffee oil based on the dry weight of the spent coffee grounds.

However, hexane is a petroleum-derived solvent with negative impacts on human health and the environment. Even though hexane is typically evaporated during the extraction process, there is a risk of residual hexane remaining in the extract and thus the obtained final product, which may not be suitable for consumption or applications in contact with skin. Thus, high efforts are required to remove the remaining hexane.

Moreover, hexane, as an unpolar solvent, does not extract polar compounds such as polyphenols from the starting material, e.g. the spent coffee grounds. Since polyphenols are reported to have many health benefits, it is desired to extract these and include these in the obtained product as well.

Thus, new techniques to extract coffee oil from biowastes, particularly spent coffee grounds, are needed to unlock their full potential.

In the art, it was previously tried to replace hexane with other solvents, which provide less or no risks on human health and the environment. However, the used solvents provided lower extraction yields (approx. 14 % of coffee oil based on the dry weight of the spent coffee grounds) than hexane and were thus not considered as suitable alternatives.

The primary object of the present invention was thus to provide improved methods for extracting coffee oil from spent coffee grounds, which overcome the above disadvantages.

The primary object of the present invention is solved by a method for extracting coffee oil from spent coffee grounds, the method comprising or consisting of the steps
i) providing spent coffee grounds,
   preferably wherein the spent coffee grounds have a water content in the range of from 50 to 80 wt.-%, preferably of from 55 to 75 wt.-%, particularly preferably of from 60 to 70 wt.-%, based on the total weight of the spent coffee grounds,
   preferably wherein the spent coffee grounds have a summed content of lignocellulose (preferably cellulose, hemicellulose and lignin) in the range of from 70 to 90 wt.-%, preferably of from 72.5 to 87.5 wt.-%, preferably of from 75 to 85 wt.-%, based on the total dry matter of the spent coffee grounds,
   preferably wherein the size of at least 85 % of the particles (d85) is in a range of from 400 to 5000 µm,
   preferably wherein the spent coffee grounds refer to coffee grounds, which have undergone a treatment with a temperature of at least 150 °C for at least 10 seconds at a pressure of at least 8 bar,
ii) inoculating the spent coffee grounds provided in step i) with one, two, three or more fungi of a strain of a genus selected from the group consisting of *Penicillium* and *Mucor,* to obtain inoculated coffee grounds,
iii) fermenting the inoculated coffee grounds obtained in step ii) for at least 24 h, to obtain a fermented composition,
iv) extracting coffee oil from the fermented composition by a method comprising a step of Soxhlet extraction and/or CO₂ extraction, to obtain a composition comprising coffee oil.

It was surprisingly found that with the method according to the invention a particularly high yield of coffee oil could be extracted (approx. 20 wt.-%, based on the dry weight of the spent coffee grounds before the extraction), which was comparable to the yields obtained with hexane extraction, however, without requiring the use of hexane. Further, the obtained yield is approximately 50 % higher than obtained with other methods, in which hexane was replaced by other solvents (approx. 14 wt.-%, dry weight of the spent coffee grounds before the extraction).

Preferably, the yields can be determined gravimetrically in wt.-% by dividing the weight of the extracted oil through the dry weight of the spent coffee grounds before the extraction.

As described above, the spent coffee grounds are coffee grounds, which have already been used in a process for producing coffee. Typically such processes apply temperatures of at least 150 °C for at least 10 seconds and a pressure of at least 8 bar.

Preferably, the temperature in these processes is at least 160 °C, further preferably at least 170 °C, particularly preferably at least 180 °C.

Preferably, the pressure in these processes is at least 9 bar, further preferably at least 10 bar, particularly preferably at least 11 bar, even further preferably at least 12 bar.

Preferably the temperature (and pressure) are applied for at least 15 seconds, further preferably at least 20 seconds, particularly preferably at least 25 seconds.

Thus, the term "spent coffee grounds" refers to a process for producing coffee as described above, i.e. with any of the described temperatures, time and pressure described above.

In some processes, coffee is brewed at much lower temperatures, e.g. at 85 °C, and the processes provide used coffee grounds as well. However, it was found that these used coffee grounds did not provided a lower yield with the method according to the invention and resulted in oil with a burnt smell, which was not or much less observed with the spent coffee grounds as defined herein.

Similarly, used coffee grounds obtained from processes with lower pressure or lower time of (temperature and/or pressure) exposure provided a lower yield with the method according to the invention and resulted in oil with a burnt smell, which was not or much less observed with the spent coffee grounds as defined herein.

Preferably, the spent coffee grounds are of the species *C. arabica* and/or *C. canephora* (robusta) or comprise spent coffee grounds of the of the species *C. arabica* and/or *C. canephora.*

It is further preferred that the spent coffee grounds provided in step i) of the method according to the invention have a particle size d10 of 410 µm, preferably of 420 µm, particularly preferably of 430 µm, further preferably of 440 µm, based on the number of particles.

The term d10 of 410 µm preferably indicates that 10 % of the particles (based on the number of particles) has a smaller average diameter (preferably diameter of an equivalent sphere) than 410 µm. The same applies accordingly to other sizes and to d90 or d50 as described herein.

It is further preferred that the spent coffee grounds provided in step i) of the method according to the invention have a particle size d90 of 4950 µm, preferably of 4900 µm, particularly preferably of 4850 µm, further preferably of 4800 µm, even further preferably of 4780 µm.

It is further preferred that the spent coffee grounds provided in step i) of the method according to the invention have a particle size d50 in the range of from 1700 to 2500 µm, preferably in the range of from 1800 to 2400 µm, particularly preferably in the range of from 1900 to 2300 µm, further preferably in the range of from 2000 to 2200 µm, even further preferably in the range of from 2050 to 2150 µm.

The term d50 in the range of from 1700 to 2500 µm preferably indicates that 50 % of the particles (based on the number of particles) have an average diameter (preferably diameter of an equivalent sphere) in said range. The average diameter of the other particles may be larger or smaller. The same applies accordingly to other ranges.

Further preferably, the content of fines in the spent coffee grounds provided in step i) of the method according to the invention is in a range of from 30 to 65 %, preferably in the range of from 35 to 60 %, particularly preferably in the range of from 38 to 55 %, wherein the content of fines refers to particles of a size of at most 2000 µm.

Preferably, the term "particle size", as used herein, is based on the number of particles.

Preferably, the term "particle size", as used herein, refers to the average diameter of the particles, preferably based on each particle separately.

Preferably, the term "the size of at least 85 % of the particles", as used herein, refers to the percentage based on the number of particles.

Preferably, the term "inoculating", as used herein, describes a step of adding the respective fungus or fungi, preferably to sterilized grounds. In case the fungus or the fungi are added to non-sterilized grounds, the term "inoculating", as used herein, preferably refers to increasing the cell density of the fungus or fungi on/in the grounds. In any case, the term describes the addition of the fungus or fungi and shall not refer to a simple growth (and thus increasing the cell density, such as by naturally occurring growth) of the fungi without such an addition. It was found that in non-sterilized grounds, the microorganisms compete for growth and expansion such that the growth and expansion of the fungi as used in the method according to the invention was not controllable and thus led to diverging, rather unpredictable results. Preferably, the spent coffee grounds provided in step i) are sterilized before the inoculation. Preferably, sterilization refers to a reduction of the number of fungi and does not require their complete absence.

Preferably, the term "sterilized grounds", as used herein, refers to grounds substantially free of microorganisms, preferably completely free of living microorganisms and with a residual amount of at most 10⁻⁶ microorganisms in one unit of grounds, which describes that in 1 million equally treated units of grounds at most 1 unit contains a (preferably one) living microorganism.

It is preferred in the method according to the invention that the cell density of the inoculated fungus in the inoculated coffee grounds (preferably at 55-60% humidity) obtained in step ii) of the method according to the invention is at least 1 × 10⁸ spores/g wet-matter. The counting of spores in the inoculum solution may be performed in a Neubauer chamber and with an optical microscope.

In case more than one fungi, as described herein, are inoculated in step ii), it is preferred that the cell density refers to the summed cell densities of all of said fungi.

In case more than one fungi, as described herein, are inoculated in step ii), it is preferred that the cell density refers to the cell density of at least one of said fungi (individually in case of more than one).

It was surprisingly found that the *Penicillium* and *Mucor* species are able to grow on the spent coffee grounds and to increase the yield of extracted coffee oil by the step of fermentation in the method according to the invention. It was found that these fungi break down the cell wall and hydrolyse the coffee oil, which increases the accessibility of the coffee oil for extraction and advantageously generates oils with self-emulsifying properties.

Further, it is preferred that the term "coffee oil", as used herein, refers to a composition comprising linoleic acid, palmitic acid and/or oleic acid. Preferably, the term "coffee oil", as used herein, refers to a composition comprising at least one of linoleic acid, palmitic acid and oleic acid. Preferably, the term "coffee oil", as used herein, refers to a composition comprising at least two of linoleic acid, palmitic acid and oleic acid. Preferably, the term "coffee oil", as used herein, refers to a composition comprising at least two of linoleic acid, palmitic acid and oleic acid.

Preferably, the term "coffee oil", as used herein, refers to a composition comprising
- 30 to 50 wt.-%, preferably 35 to 45 wt.-%, further preferably 37.5 to 42.5 wt.-% linoleic acid, and/or
- 25 to 40 wt.-%, preferably 27.5 to 37.5 wt.-%, further preferably 30 to 35 wt.-% palmitic acid, and/or
- 5 to 25 wt.-%, preferably 7.5 to 20 wt.-%, further preferably 10 to 15 wt.-% oleic acid,
each based on the total weight of the composition.

Preferably, the term "coffee oil", as used herein, refers to a composition comprising
- 30 to 50 wt.-%, preferably 35 to 45 wt.-%, further preferably 37.5 to 42.5 wt.-% linoleic acid, and
- 25 to 40 wt.-%, preferably 27.5 to 37.5 wt.-%, further preferably 30 to 35 wt.-% palmitic acid, and
- 5 to 25 wt.-%, preferably 7.5 to 20 wt.-%, further preferably 10 to 15 wt.-% oleic acid,
each based on the total weight of the composition.

It is preferred in the method according to the invention that the, one, two, three or more or all fungi in step ii) is/are selected from the group consisting of *Penicillium albocoremium, Penicillium aurantiogriseum, Penicillium bilaiae, Penicillium camemberti, Penicillium candidum, Penicillium chrysogenum* , *Penicillium claviforme, Penicillium coffeae, Penicillium commune, Penicillium crustosum, Penicillium digitatum, Penicillium echinulatum, Penicillium expansum, Penicillium funiculosum, Penicillium glabrum, Penicillium imranianum, Penicillium italicum, Penicillium lacussarmientei, Penicillium lusitanum, Penicillium purpurogenum, Penicillium roqueforti, Penicillium stoloniferum, Penicillium ulaiense, Penicillium verrucosum, Penicillium viridicatum, Mucor amphibiorum, Mucor circinelloides, Mucor ellipsoideus, Mucor fragilis, Mucor hiemalis, Mucor hiemalis f. silvaticus, Mucor indicus, Mucor mucedo, Mucor paronychius, Mucor piriformis, Mucor plumbeus, Mucor pseudolusitanicus, Mucor racemosus, Mucor ramosissimus, Mucor variicolumellatus,* and *Mucor velutinosus,*
preferably wherein the, one, two, three or more or all fungi in step ii) is/are selected from the group consisting of *Mucor hiemalis, M. circinelloides Mucor sp., Penicillium coffeae, Penicillium digitatum, Penicillium expansum,* and *Penicillium funiculosum,*
particularly preferably wherein the or one fungus in step ii) is *Penicillium funiculosum.*

It was further surprisingly found that the fungi of the genus *Penicillium* were even more active than the fungi of the genus *Mucor.* Particularly, *Penicillium funiculosum* was the most active species, typically providing the highest yields of coffee oil in the extract.

It is further preferred in the method according to the invention that the fermentation in step iii) is conducted for at least 48 h, preferably at least 60 h, further preferably at least 72 h, particularly preferably at least 96 h, more preferably at least 120 h, even further preferably at least 144 h, especially preferably at least 168 h.

Additionally or alternatively, it is preferred that the fermentation is conducted at a temperature in the range of from 12 to 30 °C, preferably of from 15 to 27 °C, further preferably of from 16 to 25 °C, particularly preferably of from 17 to 24°C, more preferably of from 19 to 23 °C.

It was advantageously found that the hydrolysis, as described above, can be controlled, since it increases almost linearly over the time and can be deduced from the duration of the fermentation. It was found that the degree of hydrolysis increases approximately 1 % per day. Thus, the fermentation can be adjusted to provide the desired degree of oil hydrolysis.

It is preferred in the method according to the invention that the, one, two, three or more or all solvent(s) in the extraction, preferably Soxhlet extraction, in step iii) is/are selected from polar solvents, preferably polar green solvents,
preferably wherein the, one, two, three or more or all solvent(s) in the extraction, preferably Soxhlet extraction, in step iii) is/are selected from the group consisting of water, ethanol, acetone, dimelthylsulfoxide (DMSO), isopropanol, methanol, ethylacetate, Methyl tert-butyl ether (MTBE), n-methylpyrrolidone.

Preferably, the Soxhlet extraction as described in the context of the present invention is a liquid-liquid Soxhlet extraction.

It is preferred in the method according to the invention that the solvent(s) in the extraction is not or do not include hexane.

It was surprisingly found in own experiments that the obtained extracts contained a particularly high amount of polyphenols, particularly if ethanol was used as the or a solvent in the extraction, compared to extracts obtained with a different method. Further, it was found that the obtained extracts were dark brown oils with a malty smell reminiscent of corn sugar syrup or molasses, if ethanol and/or acetone was used as (a) solvent in the extraction.

It is thus particularly preferred in the method according to the invention, the solvent in the extraction, preferably Soxhlet extraction, in step iii) is or comprises ethanol and/or acetone.

It is thus particularly preferred in the method according to the invention, the solvent in the extraction, preferably CO₂ extraction, in step iii) is or comprises ethanol and/or acetone.

It was further surprisingly found that the obtained extracts transparent and yellow with a low content of polyphenols and a bright and lightly ethereal smell (very similar to the extract obtained with hexane), if the extraction was or included a CO₂ extraction.

Preferably, the term "CO₂ extraction", as used herein, refers to supercritical CO₂ extraction.

Preferably, the (supercritical) CO₂ extraction is carried out at a pressure in the range of from 100 to 500 bar, preferably of from 125 to 450 bar, particularly preferably of from 150 to 400 bar.

Preferably, in step iv) of the method according to the invention, the volume ratio of CO₂ to the mixture to be extracted (e.g. the mixture obtained after step iii), the dry matter of the mixture obtained after step iii) or the mixture obtained after step iiib)) is in a range of from 2:1 to 20:1, preferably of from 5:1 to 15:1, particularly preferably of from 7.5:1 to 12.5:1, each at 400 bar.

Preferably, in step iv) of the method according to the invention, the volume ratio of CO₂ to the mixture to be extracted (e.g. the mixture obtained after step iii), the dry matter of the mixture obtained after step iii) or the mixture obtained after step iiib)) is in a range of from 7.5:1 to 25:1, preferably of from 10:1 to 20:1, particularly preferably of from 12.5:1 to 17.5:1, each at 300 bar.

Preferably, in step iv) of the method according to the invention, the volume ratio of CO₂ to the mixture to be extracted (e.g. the mixture obtained after step iii), the dry matter of the mixture obtained after step iii) or the mixture obtained after step iiib)) is in a range of from 10:1 to 30:1, preferably of from 15:1 to 25:1, particularly preferably of from 17.5:1 to 22.5:1, each at 200 bar.

Preferably, the (supercritical) CO₂ extraction is carried out using ethanol as a co-solvent, preferably wherein the volume of ethanol is in a range of from 2 to 5 % of the volume of CO₂.

Preferably, in the Soxhlet extraction, the total amount of solvent is in a range of from 2 to 10 L, preferably of from 3 to 9 L, further preferably of from 4 to 8 L, particularly preferably of from 5 to 7 L, based on 1 kg of the mixture to be extracted (e.g. the mixture obtained after step iii), the dry matter of the mixture obtained after step iii) or the mixture obtained after step iiib)).

Preferably, when the desired degree of hydrolysis is reached, the fermented composition obtained in step iii) can be dried.

It is thus preferred in the method according to the invention that the method further comprises the step
iii.b) drying the fermented composition obtained in step iii), preferably to a water content of at most 5 wt.-%.

Preferably, the fermented composition obtained in step iii) is dried until a bulk density of at least 150 kg/m³, preferably at least 175 kg/m³, particularly preferably at least 200 kg/m³.

Preferably, the drying is performed with or including a method selected from the group consisting of heat drying, sun drying, hot air drying, combined air and heat pump drying, vacuum oven drying, freeze drying, spray drying, and (vacuum) belt drying.

Preferably, the water content, as described herein, is determined by dry-loss-method or Karl-Fischer titration.

It is preferred in the method according to the invention that the method further comprises the step
v) filtering the composition comprising coffee oil obtained in step iv) to reduce the number of or to remove the fungi inoculated in step ii) and/or to reduce the amount of or to remove the coffee ground from the composition comprising coffee oil obtained in step iv),
   and/or
vi) reducing the amount of or removing the, one, two, three or more or all solvent(s) used for the extraction in step iv) from the composition comprising coffee oil obtained in step iv) or v), preferably by rotary evaporation,
   and/or
vii) reducing the amount of or removing free fatty acids from the composition comprising coffee oil obtained in step iv) or v) or vi).

The sequence of steps v), vi) and vii) may also be in a different order as described above.

In this regard, the filtration removes the fungi and/or coffee ground or reduces its number / amount from the composition obtained in the previous step. Likewise, the solvent is preferably reduced in its amount / removed from the composition obtained in the previous step.

Preferably, reducing the amount of or removing the, one, two, three or more or all solvent(s) in step vi) may be performed by increasing the temperature and/or reducing the pressure (preferably vacuum), preferably to evaporate (some of) the solvent(s).

As described herein, it is possible to obtain an advantageously high yield of coffee oil with the method according to the invention. It is thus preferred that the composition comprising coffee oil obtained in step iv), v), vi), and/or vii) comprises at least 15 wt.-% of coffee oil, based on the total weight of the composition.

Further, it is advantageous that with the method according to the invention also polyphenols can be extracted, which is very limited in the extraction with hexane. It is thus preferred that the composition comprising coffee oil obtained in step iv), v), vi), and/or vii) comprises at least 0.25 wt.-%, preferably at least 0.4 wt.-%, particularly preferably at least 0.5 wt.-%, further preferably at least 0.6 wt.-% of polyphenols, based on the total weight of the composition.

Preferably, the method according to the invention further comprises a step of bleaching, deodorizing and/or deacidifying the composition comprising the coffee oil, preferably the composition obtained after step iv), v), vi), and/or vii).

Preferably, the step of deacidification is carried out using ethanol / water mixtures (preferably with an ethanol content of from 90 to 95 wt.-%) or alkaline water. Preferably, the deacidification is carried out at temperatures in the range of from 20 to 60 °C. Preferably, the free fatty acids removed by the deacidification are used as a feedstock for further fermentation.

The present invention further relates to a composition comprising coffee oil, obtained by or obtainable by a method according to the invention.

As described above, it was surprisingly found that high yields of coffee oil could be obtained with the method according to the invention without using hexane. It is thus preferred that the amount of hexane in the composition according to the invention is at most 5 wt.-%, preferably at most 3 wt.-%, particularly preferably at most 2 wt.-%, further preferably at most 1 wt.-%, even further preferably at most 0.5 wt.-%, more preferably at most 0.1 wt.-%, even further preferably at most 0.01 wt.-%, especially preferably at most 0.001 wt.-%, most preferably at most 0.0001 wt.-%.

It was further surprisingly found that the obtained extracts contained a particularly high amount of polyphenols, if ethanol was used as (a) solvent in the extraction. Further, it was found that the obtained extracts were dark brown oils with a malty smell reminiscent of corn sugar syrup or molasses, if ethanol and/or acetone was used as (a) solvent in the extraction

It is thus particularly preferred in the composition according to the invention that it is obtained by a method according to the invention, wherein the solvent in the extraction, preferably Soxhlet extraction, in step iii) is or comprises ethanol and/or acetone.

It is thus particularly preferred in the composition according to the invention that it is obtained by a method according to the invention, wherein the solvent in the extraction, preferably CO₂ extraction, in step iii) is or comprises ethanol and/or acetone.

It was further surprisingly found that the obtained extracts were only light yellow with a low content of polyphenols and a bright and lightly ethereal smell (very similar to the extract obtained with hexane), if the extraction was or included a CO₂ extraction.

It is thus particularly preferred in the composition according to the invention that it is obtained by a method according to the invention, wherein the extraction is or includes a CO₂ extraction.

Preferably, the composition according to the invention is obtained by a method according to the invention including step iiib.), v), vi), and/or vii) as described herein.

The present invention further relates to a cosmetic product or product serving for nutrition, comprising the composition according to the invention.

Preferably, the cosmetic product according to the invention is selected from the group consisting of face creams, oils, body lotions, sunscreens, emulsions, moisturizers, lip balm/oil, eye oil, beard and hair oil, body butter, shower oil, facial/body cleansing oil, fragrance oil, soaps, and shampoos.

Preferably, the product serving for nutrition according to the invention is selected from the group consisting of cooking oil, baked goods (bread, cake and pastries), icing and frosting products, ice cream, toppings, chocolate, sauces, dips and spreads, frozen pizza, animal feed, and gummy candies.

Preferably, the cosmetic product or product serving for nutrition according to the invention comprises at least 5 wt.-%, preferably at least 10 wt.-%, particularly preferably at least 15 wt.-%, further preferably at least 20 wt.-%, more preferably at least 25 wt.-%, even further preferably at least 30 wt.-% of coffee oil, based on the total weight of the product.

It is further preferred for the cosmetic product according to the invention that the product comprises one or more further components selected from the group consisting of perfumes, stabilizers, antioxidants, preservatives, thickeners, humectants, water, natural colours / pigments, moisturisers, emollients, surfactants, UV filters.

Further aspects and advantages of the invention result from the subsequent description of preferred examples.

### Examples

### Example 1: Selection of fungi

Since the inoculated fungi need to grow on spent coffee grounds (as described herein), it was analysed, which fungi are able to colonize on spent coffee grounds.

Spent coffee grounds were obtained from a supplier, brewing coffee on industrial level.

The spent coffee grounds were cultured in Potato Dextrose Agar for 7 days at 25 °C. The formation of single colonies was observed and the colonies were isolated by dilution-to-extinction method.

The isolated colonies were analysed with macroscopic and microscopic evaluations.

Macroscopic studies were performed by studying the growth rate, colour texture and the topography of colonies using the standard medium Potato Dextrose Agar. Microscopic studies were performed by preparing a fresh slide mount with NaCl 0.9% and observed under 40x objective in a light microscope. Based on the morphology of the colonies and the microscopic images, two distinct species were observed: A fast-growing species with grey to brown aerial mycelium and a slower growing specie forming colonies with green (top of the colony) and yellow (bottom of the colony) colour. The two species were isolated and identified based on colony growth and microscopy imaging.

The capacity of both isolates to growth on different substrates as carbon and nitrogen sources was tested using the Biolog FF multiplate for Filamentous Fungi Test Panel to determine the top 10 substrates favouring the growth of the fungi.

It was thus determined that the fungi are of the *Penicillium* (*Penicillium funiculosum*) or, respectively, *Mucor* genus.

Based on the substrate utilization in the above test, the *Penicillium* sp. appeared to be the more active strain on the substrates, thus being the most promising fungi in a method for extracting coffee oil from spent coffee grounds.

### Example 2: Coffee oil extraction

Spent coffee grounds were obtained from a supplier, brewing coffee on industrial level.

The spent coffee grounds were inoculated with fungi of *Penicillium* or *Mucor* species or with a combination thereof.

The inoculated coffee grounds were allowed to be fermented by placing them in a closed container at 21 ± 2 °C for at least 2 days in static conditions. Subsequently, the fermented composition was collected, dried at 65 °C for two days and subjected to extraction with different methods.

A Soxhlet extraction using hexane as solvent (comparative example) yielded a coffee oil content of 20.1 ± 0.5 wt.-% based on the dry matter of the fermented composition.

In a parallel approach, a Soxhlet extraction using ethanol as solvent was carried out in a 2L Soxhlet extractor using an optimal ethanol to dry matter ratio of 4 : 1. The temperature in the system was very close to the boiling point of ethanol (78 °C). The solvent is cycled two times through the composition. The extraction yielded a coffee oil content of 23 ± 5 wt.-% based on the dry matter of the fermented composition.

In a parallel approach, a Soxhlet extraction using acetone as solvent was carried out in a 500 mL Soxhlet extractor, with a solvent to dry matter ratio of 3:1. The temperature in the system is very close to the boiling point of acetone (56 °C). The solvent is cycled two times through the composition. The extraction yielded a coffee oil content of approx. 22 wt.-% based on the dry matter of the fermented composition.

Both ethanol and acetone extraction yielded a dark brown oil with a malty smell reminiscent of corn sugar sirup or molasses. This is attributed to the presence of polyphenols and other secondary plant compounds.

In a parallel approach, CO₂ extraction was carried out at a pressure of 300 Bar and 60 °C as well as 150 Bar and 40 °C as a total extraction, i.e. the extraction was running for 4 h. The extraction yielded coffee oil contents in the range of from 19 to 22 wt.-% based on the dry matter of the fermented composition. The oil is transparent and yellow with a low content of polyphenols and a bright and lightly ethereal smell - overall very similar to the oil obtained with hexane.

Approaches in the prior art using other solvents than hexane, such as the solvents above, only yielded approx. 14 % coffee oil. However, it was surprisingly found that with the method according to the invention, much higher yields were obtained using the same solvents but with a previous incubation of the coffee grounds with fungi, as described herein.

### Example 3: Hydrolysis of the coffee oil

Spent coffee grounds were obtained from a supplier, brewing coffee on industrial level.

Parallel fermentation approaches were followed with the spent coffee grounds as described below. All samples were fermented at 21 ± 2 °C (RT).

The samples, which were inoculated, were first sterilized and then inoculated with 20 g of a mixture of fungi of *Penicillium* or *Mucor* species.

After the fermentation, the samples were collected and dried for 2 days at 65 °C. The content of free fatty acids was analysed by the -DGF C-V2:2020 Standard Method and is reported as g / 100 g oleic acid, wherein the coffee oil was extracted with hexane.

| **Sample** | **Inoculation** | **Duration of fermentation (days after inoculation)** | **Free fatty acids (g / 100 g oleic acid)** |
|---|---|---|---|
| **A1** | No | 0 | 12.2 |
| **A2** | | 4 | 11.4 |
| **A3** | | 7 | 12.3 |
| **A4** | | 9 | 14.4 |
| **A5** | | 14 | 18.7 |
| **A6** | | 55 | 28 |
| **B1** | Yes | 0 | 12.9 |
| **B2** | | 3 | 15.4 |
| **B3** | | 5 | 16 |
| **B4** | | 6 | 18 |
| **B5** | | 28 | 36.2 |

With the inoculation, a linear dependency of the hydrolysis of the oil was observed. Particularly, it was found that the hydrolysis already started within the first 3 days (samples B1, B2), whereas without the inoculation a similar degree of hydrolysis is not obtained before 9 days (sample 14). Further, the degree of hydrolysis obtained with the inoculation after 28 (sample B5) is not even reached after 55 days without an inoculation (sample A6).

The degree of hydrolysis not only indicates the portion of hydrolysed coffee oil, but is also a further indicator of the accessibility of the coffee oil, i.e. the expected yield of coffee oil after the extraction is higher in case of a higher degree of hydrolysis.

### Example 4: Refinements

Extracts were prepared as described in the examples above.

To remove free fatty acids from the extracts, they can be mixed with aqueous ethanol (5 wt.-% water) at room temperature at equal volumes and stirred for 10 minutes at room temperature. Then, the extract and ethanol are separated using e.g. a funnel.

Additionally or alternatively, a deodorization and clarification of dark brown oil can be carried out by adding 8 wt % of activated bentonite, 2 wt % active charcoal, stirring for 90 min at 90 °C under vacuum followed by filtration. The result is an extract with neutral smell and a light brown colour.

### Example 5: Toxicity

Samples B5 and A6 were prepared as described in Example 3. However, the samples were extracted with a Soxhlet extraction using ethanol as solvent. The obtained extracts were then tested for the presence of the mycotoxins Aflatoxine B1, Aflatoxine B2, Aflatoxine G1, Aflatoxine G2, and Ochratoxin A. A sterile non-inoculated sample kept at 4°C was used as a negative control. The mycotoxin content was below the threshold of 0.5 µg/kg in all samples.

### Example 6: Caffestol and kahweol

Samples B5 and A6 were prepared as described in Example 3, however each of the samples was extracted in three parallel approaches. In the approaches, the samples were extracted either by CO₂ extraction, by Soxhlet extraction using either acetone or ethanol as solvent.

The cafestol and kahweol contents in these samples were determined by high resolution 1H NMR at 700 Mhz.

The signals at *δ* =7.22 and *δ* =6.21 can be used to quantify cafestol and its derivatives whereas the signals at *δ* =7.27 and *δ* =6.30 are characteristic for kahweol.

Depending on the extraction methods, the following cafestol contents were determined (as weight fraction calculated from mole fraction assuming oil is 100% oleic acid):

| **Extraction method** | CO₂ | Soxhlet (acetone) | Soxhlet (ethanol) |
|---|---|---|---|
| **Cafestol content** | ~ 1 % | ~ 2.2 % | ~ 3.8 % |

Independent of the extraction method, no kahweol was detectable in the extracts. Even though the used spent coffee grounds were from the robusta variety, which is very low in kahweol, the kahweol content was surprising and even lower than expected.

### Example 7: Cosmetic formulations, moisturizing cream

A moisturizing cream was produced using 25% of hydrolysed oil of the above examples (in separate approaches), 10 % Jojoba oil and 63% of water. The mixture emulsified upon stirring at 70 °C and did not require additional surfactant. 1% of low molecular weight hyaluronic acid was added to enhance the viscosity, however the mixture was still liquid. Upon adding 1% of high molecular weight hyaluronic acid, the mixture became more solid having a favourable texture similar to a moisturizing facial cream. Adding 2 drops of lavender or vanilla oil produced of cream where the coffee oil scent was barely noticeable.

### Example 8: Cosmetic formulations, body oil

### A body oil base comprising

prunus amygdalus dulcis oil, isopropyl palmitate, simmondsia chinensis seed oil, tocopheryl acetate, spirulina platensis extract, tocopherol, helianthus annuus (sunflower) seed oil, parfum, linalool, citronellol, limonene
was provided.

Hydrolysed oils of the above examples were added in separate approaches. The optimal concentration of added coffee oil was determined sensorically, where both smell, appearance and texture of the formulations were taken into account. The optimal addition of hydrolysed coffee oil to body oil was approx. 10%. The hydrolysed coffee oil's compatibility with existing formulations was verified.

### Example 9: Cosmetic formulations, face cream

A face cream base comprising
aqua, prunus amygdalus dulcis oil, cocoglycerides, sorbitol, glycerin, polyglyceryl-3 methyl-glucose distearate, butyrospermum parkii (shea) butter, cetearyl alcohol, cera alba, ascophyllum nodosum extract, spirulina platensis extract, panthenol, xanthan gum, tocopherol, phenoxyethanol, benzoic acid, dehydroacetic acid, ethylhexylglycerin, parfum, sodium chloride, limonene, citral, sodium sulfate, sodium hydroxyde, citric acid, tartrazine (Cl 19140), brilliant blue (Cl 42090)
was provided.

Hydrolysed oils of the above examples were added in separate approaches. The optimal concentration of added coffee oil was determined sensorically, where both smell, appearance and texture of the formulations were taken into account. The optimal addition of hydrolysed coffee oil to face cream was approx. 5%. The hydrolysed coffee oil's compatibility with existing formulations was verified.

## Claims

1. Method for extracting coffee oil from spent coffee grounds, the method comprising or consisting of the steps
i) providing spent coffee grounds,
wherein the spent coffee grounds have a water content in the range of from 50 to 80 wt.-%, preferably of from 55 to 75 wt.-%, particularly preferably of from 60 to 70 wt.-%, based on the total weight of the spent coffee grounds,
wherein the spent coffee grounds have a summed content of cellulose, hemicellulose and lignin in the range of from 70 to 90 wt.-%, preferably of from 72.5 to 87.5 wt.-%, preferably of from 75 to 85 wt.-%, based on the total dry matter of the spent coffee grounds,
wherein the size of at least 85 % of the particles is in a range of from 400 to 5000 µm,
and wherein the spent coffee grounds refer to coffee grounds, which have undergone a treatment with a temperature of at least 150 °C for at least 10 seconds at a pressure of at least 8 bar,
ii) inoculating the spent coffee grounds provided in step i) with one, two, three or more fungi of a genus selected from the group consisting of *Penicillium* and *Mucor,* to obtain inoculated coffee grounds,
iii) fermenting the inoculated coffee grounds obtained in step ii) for at least 24 h, to obtain a fermented composition,
iv) extracting coffee oil from the fermented composition by a method comprising a step of Soxhlet extraction and/or CO₂ extraction, to obtain a composition comprising coffee oil.

2. Method according to claim 1, wherein the cell density of the inoculated fungus in the inoculated coffee grounds is at least 1 × 10⁸ spores/g wet-matter.

3. Method according to any of the preceding claims, wherein the, one, two, three or more or all fungi in step ii) is/are selected from the group consisting of *Penicillium albocoremium, Penicillium aurantiogriseum, Penicillium bilaiae, Penicillium camemberti, Penicillium candidum, Penicillium chrysogenum* , *Penicillium claviforme, Penicillium coffeae, Penicillium commune, Penicillium crustosum, Penicillium digitatum, Penicillium echinulatum, Penicillium expansum, Penicillium funiculosum, Penicillium glabrum, Penicillium imranianum, Penicillium italicum, Penicillium lacussarmientei, Penicillium lusitanum, Penicillium purpurogenum, Penicillium roqueforti, Penicillium stoloniferum, Penicillium ulaiense, Penicillium verrucosum, Penicillium viridicatum, Mucor amphibiorum, Mucor circinelloides, Mucor ellipsoideus, Mucor fragilis, Mucor hiemalis, Mucor hiemalis f. silvaticus, Mucor indicus, Mucor mucedo, Mucor paronychius, Mucor piriformis, Mucor plumbeus, Mucor pseudolusitanicus, Mucor racemosus, Mucorramosissimus, Mucor variicolumellatus,* and *Mucor velutinosus,*
preferably wherein the, one, two, three or more or all fungi in step ii) is/are selected from the group consisting of *Mucor hiemalis, M. circinelloides Mucor sp., Penicillium coffeae, Penicillium digitatum, Penicillium expansum,* and *Penicillium funiculosum,*
particularly preferably wherein the or one fungus in step ii) is *Penicillium funiculosum.*

4. Method according to any of the preceding claims, wherein the fermentation in step iii) is conducted for at least 48 h, preferably at least 60 h, further preferably at least 72 h, particularly preferably at least 96 h, more preferably at least 120 h, even further preferably at least 144 h, especially preferably at least 168 h, and/or
wherein the fermentation is conducted at a temperature in the range of from 12 to 30 °C, preferably of from 15 to 27 °C, further preferably of from 16 to 25 °C, particularly preferably of from 17 to 24°C, more preferably of from 19 to 23 °C.

5. Method according to any of the preceding claims, wherein the, one, two, three or more or all solvent(s) in the extraction, preferably Soxhlet extraction, in step iii) is/are selected from polar solvents, preferably polar green solvents,
preferably wherein the, one, two, three or more or all solvent(s) in the extraction, preferably Soxhlet extraction, in step iii) is/are selected from the group consisting of water, ethanol, acetone, dimelthylsulfoxide (DMSO), isopropanol, methanol, ethylacetate, Methyl tert-butyl ether (MTBE), n-methylpyrrolidone.

6. Method according to any of the preceding claims, wherein the method further comprises the step
iii.b) drying the fermented composition obtained in step iii), preferably to a water content of at most 5 wt.-%.

7. Method according to any of the preceding claims, wherein the method further comprises the step
v) filtering the composition comprising coffee oil obtained in step iv) to reduce the number of or to remove the fungi inoculated in step ii) and/or to reduce the amount of or to remove the coffee ground from the composition comprising coffee oil obtained in step iv).

8. Method according to any of the preceding claims, wherein the method further comprises the step
vi) reducing the amount of or removing the, one, two, three or more or all solvent(s) used for the extraction in step iv) from the composition comprising coffee oil obtained in step iv) or v).

9. Method according to any of the preceding claims, wherein the method further comprises the step
vii) reducing the amount of or removing free fatty acids from the composition comprising coffee oil obtained in step iv) or v) or vi).

10. Method according to any of the preceding claims, wherein the composition comprising coffee oil obtained in step iv), v), vi), and/or vii) comprises at least 15 wt.-% of coffee oil, based on the total weight of the composition.

11. Composition comprising coffee oil, obtained by or obtainable by a method according to any of the preceding claims.

12. Cosmetic product or product serving for nutrition, comprising the composition according to claim 11.

13. Cosmetic product or product serving for nutrition according to claim 12, comprising at least 5 wt.-%, preferably at least 10 wt.-%, particularly preferably at least 15 wt.-%, further preferably at least 20 wt.-%, more preferably at least 25 wt.-%, even further preferably at least 30 wt.-% of coffee oil, based on the total weight of the product.

14. Cosmetic product according to claim 12 or 13, further comprising one or more further components selected from the group consisting of perfumes, stabilizers, antioxidants, preservatives, thickeners, humectants, water, natural colours / pigments, moisturisers, emollients, surfactants, UV filters.
